# EUROPEAN PATENT SPECIFICATION

(11) **EP 2 464 406 B1**
(45) Date of publication and mention of the grant of the patent: **26.10.2016**
(21) Application number: 10808624.0
(22) Date of filing: 10.08.2010
(51) Int. Cl.: A61L 29/14, A61L 29/16, C07D 311/72, A61L 2/18

(54) **CATHETER LOCK SOLUTIONS UTILIZING TOCOPHEROL AND MID-CHAIN FATTY ACIDS**
KATHETER-LOCKLÖSUNGEN MIT TOCOPHEROL UND MITTELKETTEN-FETTSÄUREN
SOLUTIONS VERROU POUR CATHÉTER UTILISANT LE TOCOPHÉROL ET DES ACIDES GRAS À CHAÎNE MOYENNE

(30) Priority: 10.08.2009 US 232721 P
(43) Date of publication of application: 20.06.2012
(73) Proprietor: Proviflo, LLC, Clinton, Mississippi 39060 (US)
(72) Inventor: FINCH, Charles, David, Jr., Clinton Mississippi 39056 (US)
(74) Representative: Hamer, Christopher K.
(86) International application number: PCT/US2010/045011
(87) International publication number: WO 2011/019710

(56) References cited:
- WO-A1-2005/123149
- WO-A1-2006/036557
- WO-A2-2006/036970
- US-A1- 2003 175 323
- US-A1- 2005 043 673
- US-A1- 2006 058 737
- US-A1- 2006 253 063
- US-B1- 6 958 049

## Description

### BACKGROUND OF THE INVENTION

### 1. Field of the Invention

The present invention relates to compositions and kits for locking the lumen of an implanted catheter between successive uses.

Implanted catheters, including extravascular and intravenous catheters, are employed in numerous medical procedures. For example, extravascular catheters, including Foley catheters, are commonly used to drain urine and other body fluids. Intravenous (IV) therapy relies on long-term implantation of a venous catheter to deliver fluids, medications, and other substances to a patient. A peripherally inserted central catheter (PICC or PIC line) is a common form of intravenous access. Hemodialysis and hemofiltration (also referred to as haemofiltration), both rely on separate draw and return catheters implanted in a vein and/or artery to allow extra corporeal treatment of the blood. Peritoneal dialysis, in contrast, relies on a single catheter implanted in the peritoneum to permit introduction and withdrawal of dialysate to permit *in situ* dialysis.

The need to leave catheters implanted for a prolonged period raises a number of concerns. First, the catheters can become infected requiring treatment of the patient and often requires removal of the catheter. This is a particular problem with transcutaneous catheters where the skin penetration is a common route of infection. Urinary catheterization exposes patients to increased risk of urinary, kidney and blood (sepsis) infections. Some other catheter-related infectious complications include septic shock, endocarditis, septic arthritis, osteomyelitis and epidural abscess. Biofilms of infectious bacteria and yeasts often colonize indwelling catheters. Second, implanted catheters can often become plugged or fouled over time. Catheter malfunction is often due to extrinsic and/or intrinsic thrombosis, and has been found to be the most common indication for catheter removal. This is a particular problem with intravascular catheters where clotting and intrinsic thrombus formation (i.e. within the catheter lumen) can be problematic. Extrinsic thrombosis, including central venous thrombosis, is also an important and common complication.

To reduce problems associated with thrombus formation, it is now common to "lock" intravascular access catheters between successive uses. Locking typically involves first flushing the catheter with saline to remove blood, medications, cellular debris and other substances from the catheter lumen. After the catheter has been flushed, a locking solution, typically heparin, is then injected to displace the saline and fill the lumen. The heparin locking solution both excludes blood from the lumen and actively inhibits clotting and thrombus formation within the lumen. To address infection, various antimicrobial substances have been combined with the locking solution in order to inhibit infection at the same time that thrombosis is being inhibited. However, problems with current and continuously emerging resistance to antimicrobial substances, as well as the over-use (and hence the increased risk of developing resistance) of anti-microbials, is an ever-growing concern.

While generally effective, the use of heparin locks suffers from a number of problems and disadvantages. For example, some thrombi may still form at the distal tip of the catheter despite the use of heparin. The need to prepare a heparin solution at the end of every catheter treatment session is time-consuming and presents an opportunity for error by a caregiver. Additionally, heparin has been shown to stimulate biofilm formation which makes it necessary to combine an antimicrobial compound in the heparin lock solution. Heparin is also associated with potentially adverse effects, including heparin-induced thrombocytopenia and bleeding risks.

Various acids have been proposed for use as antimicrobial catheter lock solutions. However, high concentrations of these acids have been shown to cause hemolysis of red blood cells and other harmful effects. Citrate, an ionic form of citric acid, will chelate the divalent cations including the calcium ions in blood and tissue. Serious symptoms have been reported when the ionized calcium blood level decreases. Spillage of extra locking solution into the patient includes miscalculating the lock volume, multiple instillations of solution into the same lumen and even deliberate over injection of solution to clear an occluded catheter. Thus, there is legitimate concern that risks of using concentrated sodium citrate for a catheter lock are not well understood.

For at least these reasons, it is desirable to provide improved methods, compositions, and kits for locking implanted catheters between successive uses. Such locking methods should inhibit fouling of the catheter lumen and/or reduce the chance of infection, preferably both. In particular, such methods should be cidal against a broad spectrum of microorganisms and discourage the development of resistant microbes without damaging blood and/or tissue cells. The lock should be relatively inexpensive, non-toxic, easy to store, compatible with the catheter material, safe if inadvertently infused systemically, easy to implement, require minimum or no preparation, and be useful with most or all types of implanted catheters, including hemodialysis and hemofiltration catheters, IV catheters, peritoneal dialysis catheters, urinary catheters, chemotherapy catheters, and the like. At least some of these objectives will be met by the invention described hereinafter.

### 2. Description of the Background Art

U.S. Patent Publication No. 2008/0118544 teaches drug release coatings for medical devices that may include lauric acid and argatroban. U.S. Patent Publication No. 2007/0292355 teaches an anti-infection composition that can include lauric or caprylic acid and triolein. U.S. Patent Publication No. 2006/0062850 describes a controlled release composition for a catheter that includes caprylic, capric, or lauric acid. U.S. Patent Publication No. 2006/0052757 teaches an implantable catheter that has a layer of bioactive material including argatroban. U.S. Patent Publication No. 2005/0181008 describes drug-coated or drug-impregnated implantable devices wherein the composition may include argatroban. U.S. Patent Publication No. 2006/0257390 teaches an instance of citrate injected as a catheter lock. U.S. Patent Publication No. 2006/0177477 teaches a catheter lock solution comprising citrate and a paraben. U.S. Patent Publication No. 2006/0094690 describes the use of a catheter locking solution comprising taurolidine in combination with citrate and citric acid. U.S. Patent No. 6,958,049 explains the use of a catheter lock solution including a citrate salt. U.S. Patent No. 6,423,706 describes an antimicrobial catheter lock solution including citric acid. U.S. Patent Nos. 6,679,870 and 6,685,694 describe locking solutions for catheters using a lower alcohol and sodium citrate or citric acid. U.S. Patent No. 4,929,242 describes a solution containing glycerol and having a density similar to that of blood for providing a heparin lock on an intravenous catheter. U.S. Patent No. 5,077,281 describes an antimicrobial solution containing a taurolin compound for inhibiting coagulation in dialysis catheters and other vascular prostheses. PCT Publication No. WO 00/01391 describes an antimicrobial lock comprising a taurinamide derivative. Citrate has been discussed as a locking solution in various concentrations or in combination with other compounds in numerous publications including, for example, Ash et al., ASAIO Journal, (2000) 46(2):222; Mandolfo et al., Journal of Vascular Access, (2006) 7(3):99-102; Dogra et al., Journal of the American Society of Nephrology,(2002) 13(8):2133-2139; and Meeus et al., Blood Purification (2005) 23(2):101-105.
WO 2005/123149 describes a lock solution for medical devices such as catheters. The lock solution comprises carbohydrates and/or glucose degradation products. The lock solution may further comprise additives, eg. vitamins such as alpha-tocopherol, and may further comprise viscosity enhancing additives, eg. lipids, to avoid bleeding out of the lock solution.

### SUMMARY OF THE INVENTION

In a first aspect of the present invention, a kit for locking an implanted catheter according to Claim 1. Also disclosed herein is a method for locking an implanted catheter comprising filling a lumen of an implanted catheter that opens to a body lumen with a hydrophobic antimicrobial lock solution comprising a tocopherol compound and at least one saturated mid-chain fatty acid (MCFA). The lock has a viscosity sufficient to inhibit loss from the catheter lumen to the body lumen. Preferably, the lock has a viscosity greater than about 3 milliPascal seconds (mPa.s). Mid-chain (i.e. medium chain) fatty acids include any carboxylic acid with an aliphatic chain of 8-14 carbon atoms. Saturated fatty acids do not contain any double bonds or other functional groups along their molecular chain. The term "saturated", as defined herewith, are straight-chained molecules in which all carbons, apart from the carboxylic acid [-COOH] group, contain as many hydrogen atoms as possible.

Preferably, the tocopherol compound comprises at least one tocopherol selected from the group consisting of alpha-tocopherol, tocopherol acetate and tocopherol acid succinate ester. More preferably, alpha-tocopherol(α tocopherol or alpha-Toc) is used with a minimum viscosity of 3-4 milliPascal seconds (mPa.s), which is a viscosity greater than blood, prior to combining with the mid-chain fatty acid(s). Tocopherol is isolated from wheat germ oil. Tocopherol is a fat-soluble antioxidant that also possess antiplatelet and anticoagulant properties. Tocopherols are a class of chemical compounds of which many have vitamin E activity. Alpha-tocopherol is the most active form of vitamin E and has the most potent antiplatelet and anticoagulant activity. All of the various derivatives with vitamin activity may correctly be referred to as "vitamin E". Examples of specific forms may include, but are not limited to, alpha-tocopherol, tocopherol acetate and tocopherol acid succinate ester.

There are numerous other suitable tocopherol compounds which vary in form and chemical structure. Some of these forms are shown below, for example:

### TOCOPHEROL (Toc) Chemical Structure and Homologues

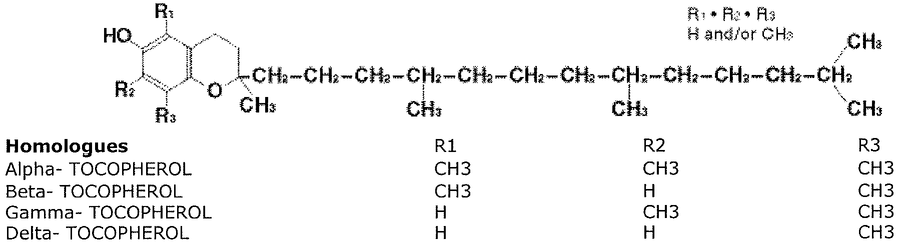

The major consideration in substituting or adding another tocopherol would be to increase the viscosity of the formulation while maintaining its essential functions as a lock. However, the advantages of using alpha-tocopherol as a component for a locking solution are specified in this document. For example, in the case of alpha-tocopherol, we have discovered that it mitigates the toxicity issues of mid-chain fatty acids, particularly octanoic acid toxicity, while solubilizing one or more of the fatty acids at physiological temperatures. Tocopherol is also particularly important in determining the cumulative total viscosity of the final solution because it is a relatively large molecule compared to octanoic (C₈H₁₆O₂) acid, decanoic (C₁₀H₂₀O₂) acid or dodecanoic (C₁₂H₂₄O₂) acid, for example. By increasing the viscosity, the persistence of the solution in the catheter lumen can be enhanced. Furthermore, a lock more viscous than blood prevents blood from entering the catheter lumen.

The mid-chain fatty acids used in the lock solutions of the present invention are selected from the group consisting of lauric acid (i.e dodecanoic acid), capric acid (i.e. decanoic acid), caprylic acid (i.e. octanoic acid) and may be used individually or in any combination. Preferably, the final lock will have a viscosity greater than 3-4 milliPascal seconds (mPa.s) which is a viscosity greater than that of blood and contain various percentages by weight or volume of at least one mid-chain fatty acid(s) and with the remaining portion including the tocopherol compound. Preferred fatty acids are octanoic acid, decanoic acid and dodecanoic acid. Octanoic and decanoic acids are liquids at the temperatures to be used for humans when mixed with the tocopherol liquid. In contrast, dodecanoic acid is a solid which must be weighed prior to dissolving the compound in either one or both of octanoic acid or decanoic acid. Optimal concentrations are easily achieved by dissolution of dodecanoic acid in octanoic acid prior to adding tocopherol. Once combined, they remain in solution at physiological temperatures. Subsequently the final formulation will be adjusted to the final volume with the tocopherol. The concentration of tocopherol may be particularly important to mitigate the specific toxicity of octanoic acid at relatively high concentrations. The tocopherol and the mid-chain fatty acid(s) establish an optimal viscosity range for effectively locking catheters.

We have discovered that combinations of these fatty acids, when used with tocopherol in a locking solution, effectively provide a stable formulation that safely inhibits microbial infection. The ability to inhibit clot formation within the lumens of implanted catheters without the need to prepare and use heparin solutions or coumarin class drugs, such as warfarin, is a significant advantage. Multiple drug interactions, allergic reactions, bleeding, and other problems associated with anticoagulant drugs like heparin and warfarin are also avoided. Moreover, mid-chain fatty acids, long used to preserve food, have the additional ability to inhibit microbial and yeast growth. Some fatty acids have been shown to inhibit the cellular intrusion and proliferation of lipid-coated viruses, including influenza. For example, dodecanoic acid, commonly found in coconut oil, is one of the most potent antimicrobial and anti-viral compounds in this category. Dodecanoic acid has greater antiviral activity than either decanoic acid or decanoic. Using a metabolizable mid-chain fatty acids is nontoxic and may also provide nutritional and antioxidant value to the patient if infused into their tissues and bloodstream. Thus, both the reduction of catheter fouling and the inhibition of infection can be achieved with the use of commonly available, widely accepted compounds and agents which are introduced to the catheter lumen in a convenient fashion, as described in more detail below.

It has been observed that tocopherol protects both internal and external body tissues from potential injury which may otherwise be caused by application of mid-chain fatty acids alone. For example, tocopherol is a capable anticoagulant and antiplatelet additive that offsets the platelet activating properties of mid-chain fatty acids. Tocopherol also enhances the emollient effects on topical application of fatty acids. Given the discovery that combinations of these fatty acids, when used with tocopherol, effectively inhibit or prevent microbial infections, it is appreciated that topical application of the lock solution could be further used to treat skin, eye or ear infections and wounds, for example.

In a second aspect of the present invention, a composition includes a hydrophobic antimicrobial catheter lock solution comprising a tocopherol compound and one or more fatty acids according to Claim 8. Preferably, alpha-tocopherol in a concentration of 0% to 50% (volume to volume) is used. However, alternative types and sources of tocopherols may be employed, which may include one or more of any of the numerous types of tocopherol compounds previously described, for
example. The fatty acids include at least one of octanoic acid (up to 50% volume/volume), decanoic acid (up to 25% volume/volume), and/or dodecanoic acid (up to 25% weight/volume). The combined formula after dissolving and mixing the chosen mid-chain fatty acid(s) is adjusted to a final volume so the combination of the tocopherol and acid(s) has a viscosity greater than 3 mPa.s.

The hydrophobic antimicrobial catheter lock solution may further comprise at least one essential oil. The oil is mixed with a fatty alcohol corresponding to at least one saturated mid-chain fatty acid. The essential oil may be oil of orange and the fatty alcohol may be octanol.

The kit of the present invention may further comprise a package, such as a box, tray, tube, envelope, pouch, or the like, for holding the container. The mid-chain fatty acid may be any combination of octanoic acid, decanoic acid and dodecanoic acid. The preferable tocopherol is alpha-tocopherol. However, alternative types and sources of tocopherol compounds may be used, which may include one or more of any of the numerous types of tocopherol compounds previously described. The volume of the solution in the container is typically in the range from 1ml-20ml (milliliters), preferably from 2ml-10ml, usually being about 2ml-4ml. Optionally, the container will usually comprise a syringe, or device to permit direct introduction of the solution into the indwelling catheter.

In a third aspect of the present invention, a hydrophobic antimicrobial solution according to Claim 12 for use in a method for disinfecting an implanted catheter includes introducing the hydrophobic antimicrobial solution into a lumen of a catheter where at least a portion of the catheter is sufficiently porous to permit diffusion of the solution outwardly from the lumen to the outer surface of the catheter and into tissues or the bloodstream surrounding the catheter to inhibit infection. The implanted catheter may be a subcutaneous or transcutaneous indwelling catheter. The antimicrobial solution is made of a tocopherol compound and at least one saturated mid-chain fatty acid. Preferably, the tocopherol is alpha-tocopherol. However, alternative types and sources of tocopherol compounds may be used, which may include one or more of any of the numerous types of tocopherol compounds previously described. The acids include at least one selected from octanoic acid, decanoic acid and dodecanoic acid.

In an aspect of the disclosure, a hydrophobic antimicrobial catheter lock solution includes alpha-tocopherol, in a concentration of 0% to 50% by volume/volume and mid-chain fatty acids. The fatty acids may include any combination of octanoic acid (0% to 25% volume/volume), decanoic acid (0% to 12.5% volume/volume), and/or dodecanoic acid (0% to 12.5% weight/volume). The combined formula after dissolving and mixing the chosen mid-chain fatty acid(s) is adjusted to a final volume with the alpha-tocopherol so that the solution has a viscosity greater than about 3 mPa.s. The lock has a viscosity in the range of about 3-5 mPa.s. Dodecanoic acid is a solid soluble in octanoic acid, decanoic and tocopherol. Concentrations of dodecanoic acid of less than about 25% dissolve readily without heating (i.e. 25% is approximately the saturation point of dodecanoic acid at room temperature). Optimal concentrations are most easily achieved by dissolution of dodecanoic acid in octanoic acid prior to adding the tocopherol. Of course, it is also possible to mix any or all of the liquid ingredients (i.e. octanoic acid and/or decanoic acid and/or tocopherol) together first and secondarily dissolve the dodecanoic acid. Alternatively, dodecanoic acid could be mixed with any of the liquids and the remaining liquid components added depending on the concentration of dodecanoic acid. Accordingly, tocopherol is unnecessary as a solvent but has important anti-platelet and anticoagulant properties necessary for the lock solution.

The lock solution further comprises at least one essential oil and at least one fatty alcohol corresponding to at least one saturated mid-chain fatty acid. The alcohol is mixed with the oil to provide an aromatic property to the lock solution.

The ability to inhibit or prevent infection of the implanted catheter can be improved by utilizing catheters where at least a portion of the catheter body is sufficiently porous to allow the lock solution to permeate the catheter body and, preferably, pass outwardly (i.e. seep, ooze, leak, diffuse) into the tissue region surrounding the catheter. While the use of such porous or partially porous catheter bodies can be beneficial with many antimicrobial locking solutions, such as those taught in U.S. Patent Nos. 4,186,745; 4,767,400; 4,968,306; 5,077,281; 5,800,392; 5,913,856; 6,949,087; 7,004,923; and U.S. Patent Publication Nos. 2006/0074388 and 2006/0253101, it is particularly useful with the acids of the present invention. It will be appreciated that mid-chain fatty acids have molecular weights and other qualities which enable them to readily penetrate into and through many porous materials. Exemplary porous materials for construction of the catheter body include silicone rubber, expanded PTFE (e.g., GORE-TEX^{®}, medical membranes), Teflon^{®} films, natural, regenerated or semi-synthetic cellulosic materials such as cellulose acetate, cellulose diacetate, cuprophane, and the like. Such materials may be formed into the tubular catheter bodies or may be incorporated as separate component(s) into the catheter bodies.

It is further contemplated that adding at least one essential oil to the lock solution improves the scent of the solution without interfering with the efficacy of the solution. An essential oil is defined herewith as a concentrated, hydrophobic liquid containing volatile aroma compounds obtained from a plant. Examples of these compounds may include oils derived from flowers, seeds, bark, wood, leaves, peel, resin, rhizome or root. More specifically, oil of orange, oil of lemon or a mixture of citrus oils may be used. Of course, in addition to these specific examples, many other essential oils could also be used. To add the essential oil to the lock solution, the oil is mixed with the alcohol corresponding to the fatty acid. The alcohol solubilizes the oil. Octanol, the straight chain fatty alcohol of octanoic acid can be used as a solvent for the essential oil. Of course, decanol (corresponding fatty alcohol of decanoic acid), dodecanol (corresponding fatty alcohol of dodecanoic acid) or any other alcohol corresponding to a fatty acid could also serve as a suitable solvent.

In another aspect of the present disclosure, a method for locking an implanted catheter comprises filing a lumen of an implanted catheter that opens to a body lumen with a hydrophobic antimicrobial lock solution comprising a tocophenol compound and at least one saturated mid-chain fatty acid (MCFA). The lock has a viscosity sufficient to inhibit loss from the catheter lumen to the body lumen. Further, at least one essential oil is mixed with a fatty alcohol that corresponds to at least one saturated mid-chain fatty acid to improve the aroma of the lock solution. The essential oil may be orange oil and the fatty alcohol may be octanol.

### BRIEF DESCRIPTION OF THE DRAWINGS

Figs. 1A and 1B illustrate methods according to the present invention for locking and disinfecting a transcutaneous catheter.
Figs. 2A-2C illustrate methods according to the present invention for locking and disinfecting a subcutaneously implanted catheter.
Figs. 3A-3C illustrate methods according to the present invention for locking and disinfecting a peritoneal dialysis catheter.
Fig. 4 illustrates a preferred aspect of the present invention where an antimicrobial locking fluid permeates into an implanted catheter body and preferably into the tissue surrounding the catheter body.
Fig. 5 illustrates a kit constructed in accordance with the principles of the present invention.

### DESCRIPTION OF THE SPECIFIC EMBODIMENTS

The present invention provides improved compositions and kits for the locking and disinfecting of subcutaneously and transcutaneously implanted catheters. The words "implanted", "subdermal", "subcutaneous" and "indwelling" are used synonymously to refer to catheter placement. These implanted catheters typically will have a distal end which is at least partially open to a body lumen. Most commonly, the catheters will be intravascular catheters where the distal end is implanted in or attached to a blood vessel, usually a vein, but in some cases an artery. Exemplary intravascular catheters include hemodialysis and hemofiltration catheters, intravenous catheters, and the like. Intravenous catheters can be used for a wide variety of purposes, including fluid infusion, drug delivery, and the like. Catheters attached other than to the vasculature include peritoneal dialysis catheters which are open to the peritoneal cavity, urinary catheters which open to the bladder, and the like.

The catheters which are treated by the lock solutions of the present invention may be transcutaneously implanted or subcutaneously implanted. By "transcutaneously implanted," it is meant that the distal end of the catheter is attached to or implanted within a target body lumen and a proximal end of the catheter is located externally to the patient. An intermediate portion of the catheter will thus pass through or penetrate the patient's skin, and the proximal end of the catheter will usually have a hub to permit selective attachment of infusion tubes, syringes, solution bags, and the like. Most commonly, the proximal attachment hub will have a luer fitting. By "subcutaneously implanted," it is meant that the entire catheter is implanted beneath the skin and no portion of the catheter extends through the skin. Such subcutaneously implanted catheters are typically attached to a fully implanted hub at their proximal ends. The hub permits percutaneous access via a needle or other penetrating element.

Before a catheter treatment session, it is standard practice to remove any residual locking solution from the catheter lumen. This process is commonly known as "flushing" the catheter. The method usually includes filling a fluid handling device, preferably a syringe, with an aliquot of normal saline or other solution sufficient to perform a catheter flush procedure. The practitioner attaches a syringe to the target intravascular catheter and draws back to remove any locking solution and discards. You then connect the syringe with the normal saline to the target intravascular catheter that requires flushing and dispenses the flushing solution into the catheter. The frequency of the catheter flush procedure may be once daily or, for particular situations, more or less frequently. After a treatment session is finished, the needle or other penetrating element is removed.

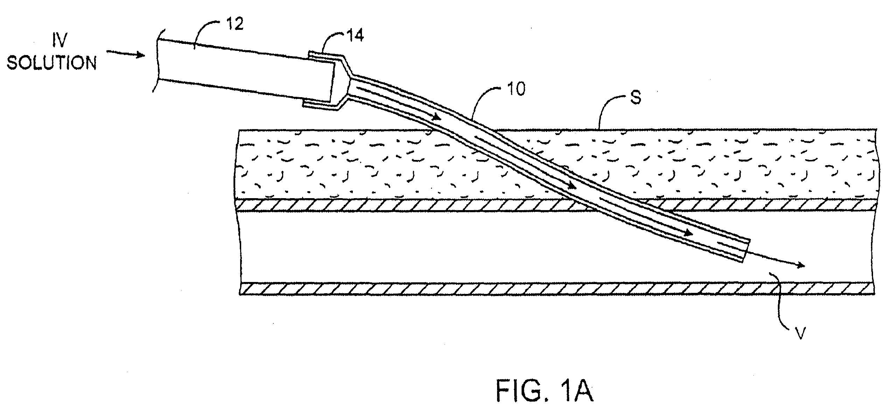

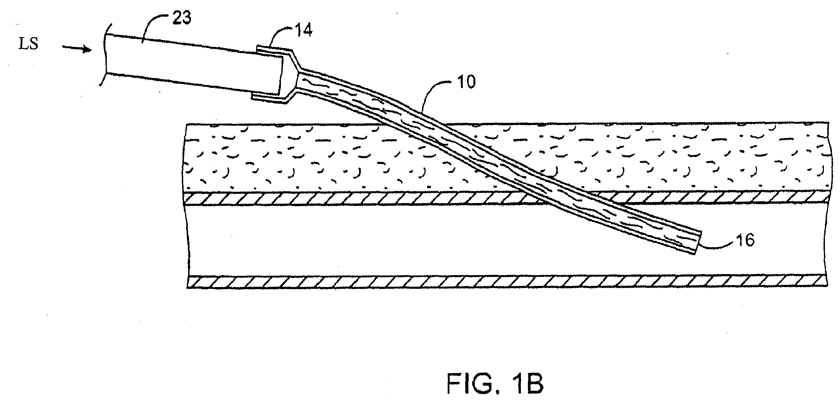

Referring now to Figs. 1A and 1B, a method for locking an implanted venous catheter 10 will be described. The venous catheter 10 will be implanted through a patient's skin S into a vein V for infusion of the patient. When it is desired to disconnect the patient from the source of infusion, it will be necessary to lock the catheter to inhibit plugging and fouling caused by coagulation, and preferably to further inhibit or eliminate the risk of infection. Shown in Fig. 1A, a tube 12 containing an IV solution will normally be connected to the proximal hub 14 of the catheter 10. The IV line 12 will be disconnected, and the catheter 10 rinsed with a flushing solution. After the flushing is completed, a lock solution (LS) of a mid-chain fatty acid(s) and tocopherol can be introduced to fill the inner lumen of the catheter 10, as shown in Fig. 1B. Usually, a sufficient volume of the lock solution LS will be introduced to completely fill the lumen of the implanted catheter 10, with minimum excess passing from distal end 16 of the catheter. The loss of excess solution into a blood vessel or most other body lumens, however, will generally not be a problem. The "column" of the solution will then occupy the inner lumen, and the proximal hub 14 will be sealed, helping retain the solution in place. It has been found that the solution of mid-chain fatty acid(s) and tocopherol will effectively inhibit clotting and coagulation at the distal end 16 as well as inhibit or eliminate infection throughout the catheter. When it is desired to reattach the patient to the IV source, the solution will be removed and the catheter lumen flushed.

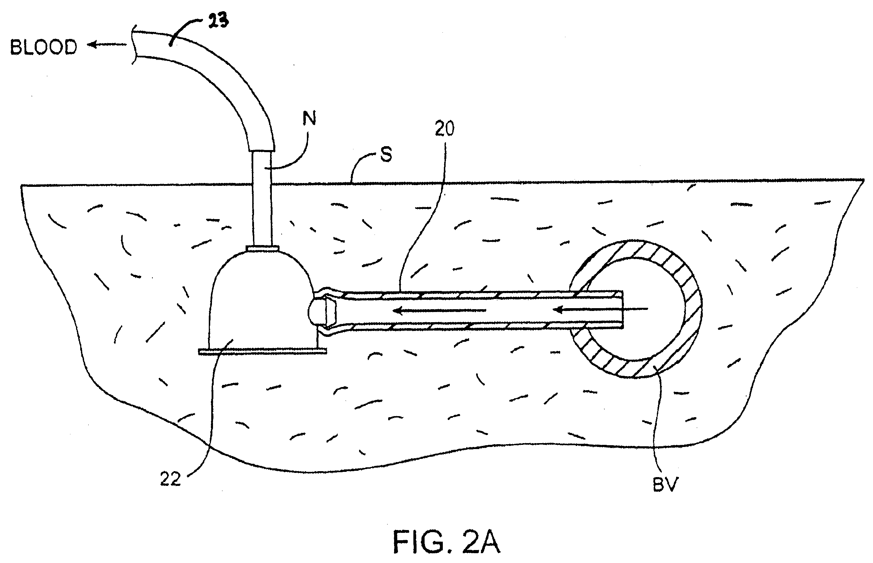

Referring now Figs. 2A-2C, locking of a subcutaneously implanted catheter 20 used for hemodialysis access will be described. The catheter 20 is implanted between a target blood vessel BV, typically a vein, and an implanted port 22. During hemodialysis, blood may be withdrawn through the catheter 20, through the port 22 and externally through a needle N and connecting line 23 used to percutaneously access the port 22 (Fig. 2A). Alternatively, the port and catheter could be used to return treated blood to the patient.

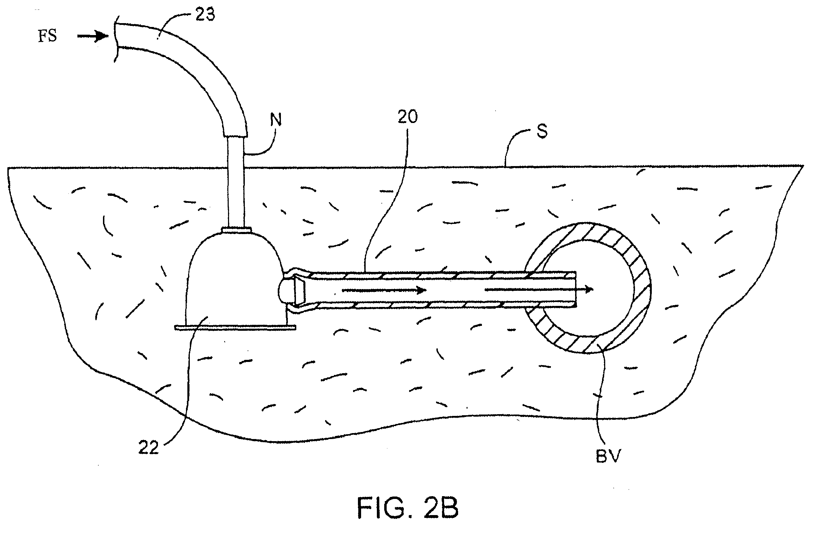

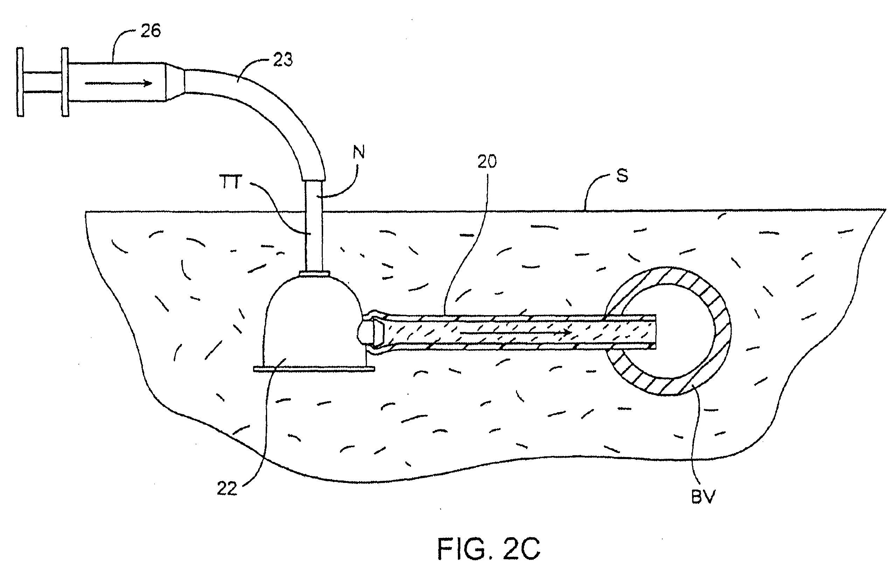

When it is desired to end a hemodialysis (or hemofiltration) treatment, a flushing solution FS will be introduced through the needle N (typically from a syringe which is attached to the connecting line 23) to flush the lumen, as depicted in Fig. 2B. After the flush is complete, a container such as syringe 26 containing the hydrophobic antimicrobial lock solution of mid-chain fatty acid(s) and tocopherol is injected through the line 23/port 22 and into the lumen of catheter 20 to displace the flushing solution and lock the catheter (Fig. 2C). The lock solution will remain in place within the catheter 20.

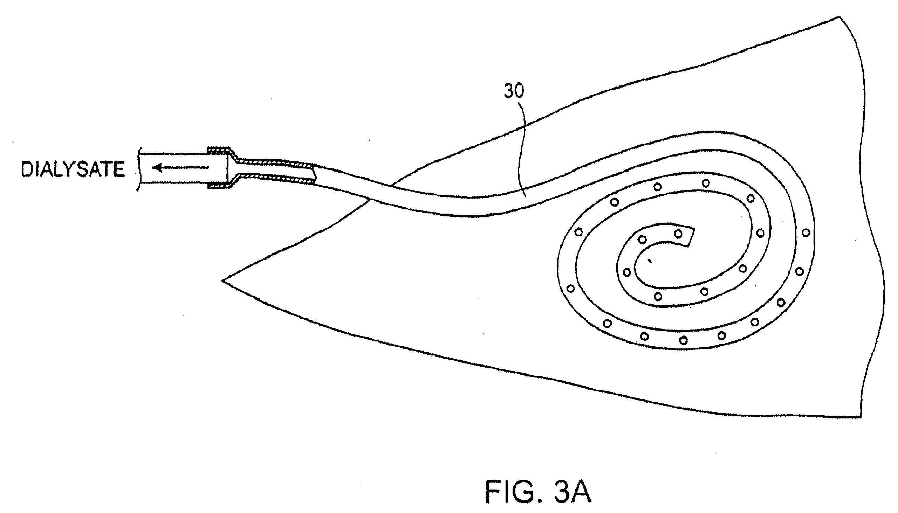

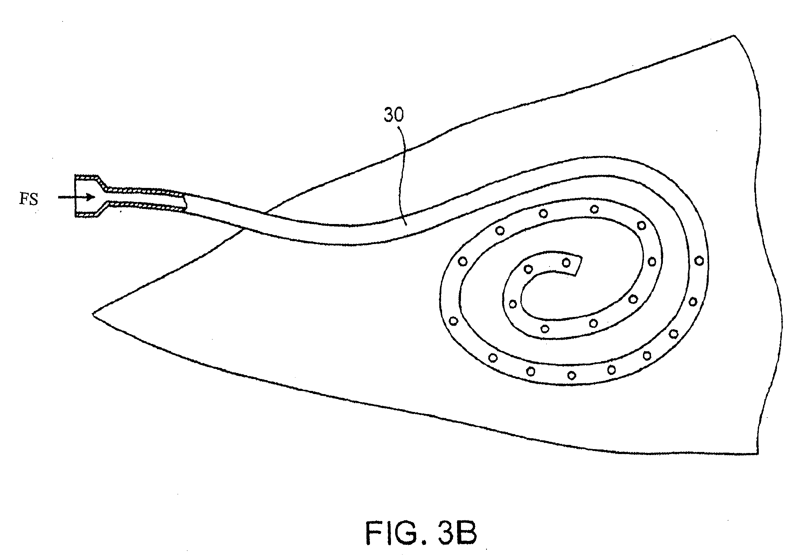

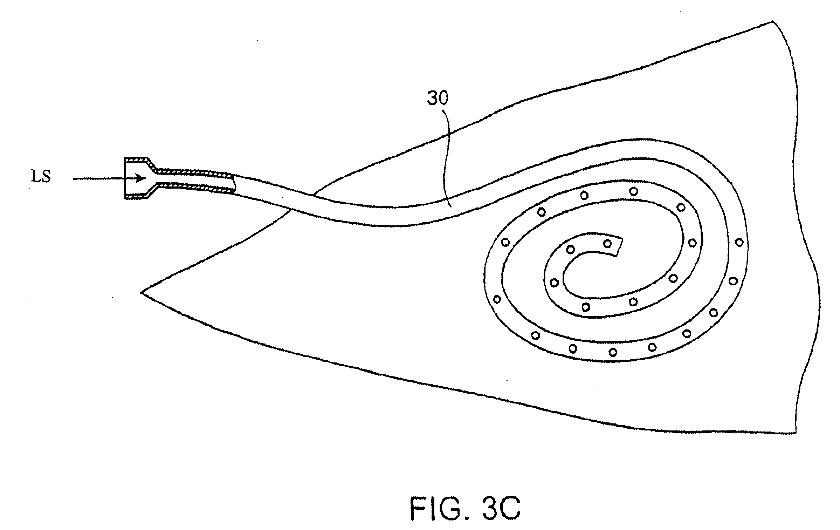

The lock solutions of the present invention may also be used to lock non-vascular catheters, such as peritoneal dialysis catheters 30, as shown in Figs. 3A-3C. After a peritoneal dialysis treatment, the used dialysate will be withdrawn from the catheter 30, as shown in Fig. 3A. After the dialysate has been sufficiently removed, the dialysis catheter 30 may optionally be flushed with a flushing solution FS, as shown in Fig. 3B. After flushing, the lock solution LS of mid-chain fatty acid(s) and tocopherol is introduced to the peritoneal dialysis catheter 30, as shown in Fig. 3C, so that it fills the lumen of the catheter, as described previously with the vascular catheters. The use of the aforementioned lock solution for peritoneal dialysis catheters is particularly advantageous in inhibiting or eliminating infections.

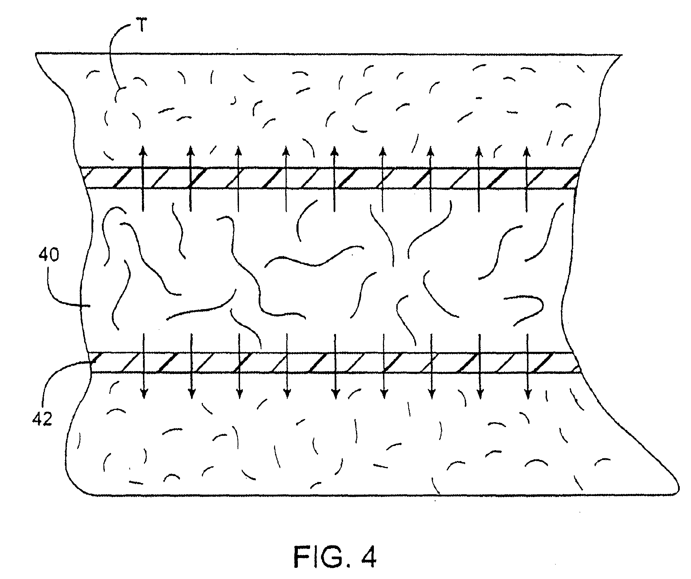

Referring now to Fig. 4, the use of a lock solution containing mid-chain fatty acid(s) and tocopherol possessing desirable antimicrobial and anticoagulant properties for locking a catheter can be enhanced by utilizing an implanted catheter which is formed at least partly from a porous material. When the lumen 40 of the porous catheter body 42 is filled with a hydrophobic antimicrobial lock solution of mid-chain fatty acid(s) and tocopherol, the solution will be able to slowly penetrate (i.e. seep) into the catheter body and preferably outwardly into the tissue T surrounding the catheter, as shown by the arrows in Fig. 4. Thus, the antimicrobial properties of the lock solution will not be entirely limited to the interior lumen of the catheter, but will also be effective on the surface of the catheter and in the tissue region immediately surrounding the catheter body. Particularly suitable materials and porosity properties for the catheter bodies have been set forth above.

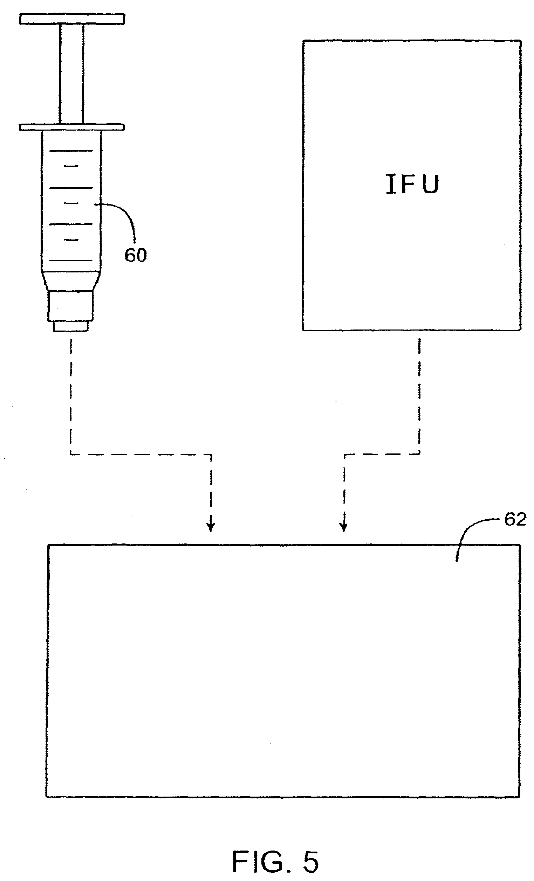

Referring now to Fig. 5, kits according to the present invention will comprise at least a container 60, such as a syringe, for holding a volume of the lock solution of the mid-chain fatty acid(s) and tocopherol and an implantable catheter lumen to receive the solution. The volume will typically be within the ranges set forth above. The kits will further contain a package 62 to hold the container 60. The package can be any conventional medical device package, including boxes, tubes, envelopes, trays, pouches and the like. In addition, the kit may contain instructions for use (IFU) setting forth a method for locking and/or disinfecting an implanted catheter by introducing the solution from the container into a lumen of the implantable catheter between successive uses of the catheter.

The invention has been described and specific examples of the invention have been portrayed. The use of those examples are not intended to limit the invention in any way. Additionally, to the extent that there are variations of the invention, which are equivalent to the inventions found in the claims, it is intended that this disclosure will cover those variations as well. For example, ingredients, such as fragrances, preservatives, coloring agents, emollients and the like, could be optionally added to the disclosed lock solution. While the above is a complete description of the preferred embodiments of the invention, various alternatives, modifications, and equivalents may be used. Therefore, the above description should not be taken as limiting the scope of the invention which is defined by the appended claims.

## Claims

1. A kit for locking an implanted catheter, said kit comprising:
a container holding a volume of a solution comprising:
a) a tocopherol compound; and
b) at least one saturated carboxylic acid with an aliphatic chain of 8-14 carbon atoms; and
an implantable catheter lumen which receives the solution.

2. The kit of claim 1, wherein the tocopherol compound comprises at least one tocopherol selected from the group consisting of alpha-tocopherol, tocopherol acetate and tocopherol acid succinate ester.

3. The kit of claim 2, wherein the tocopherol compound is alpha-tocopherol.

4. The kit of claim 3, wherein the alpha-tocopherol has a minimum viscosity of 3 milliPascal seconds prior to combining with the at least one acid.

5. The kit of claim 1, wherein the at least one acid is selected from the group consisting of octanoic acid, decanoic acid and dodecanoic acid.

6. The kit of claim 1, further comprising at least one essential oil, wherein the oil is mixed with a fatty alcohol that corresponds to at least one saturated carboxylic acid with an aliphatic chain of 8-14 carbon atoms to improve the aroma of the lock solution.

7. The kit of claim 6, wherein the at least one acid is octanoic acid or decanoic acid or dodecanoic acid or any combination thereof.

8. A hydrophobic antimicrobial catheter lock solution configured for inhibiting infection and blood clot formation to lock a lumen of an implanted catheter, the catheter open to a body lumen of a patient, the solution comprising:
a tocopherol compound combined with at least one of the following acids:
octanoic acid, up to 50% by volume/volume;
decanoic acid, up to 25% by volume/volume; and
dodecanoic acid, up to 25% by weight/volume;
wherein the combination of the tocopherol and the at least one acid provides a viscosity to the solution greater than 3 milliPascal seconds.

9. The lock solution of claim 8, wherein the tocopherol compound is alpha-tocopherol at a concentration of up to 50% (volume/volume).

10. The lock solution of claim 8, further comprising at least one essential oil, wherein the oil is mixed with a fatty alcohol corresponding to at least one saturated carboxylic acid with an aliphatic chain of 8-14 carbon atoms.

11. The lock solution of claim 10, wherein the essential oil is oil of orange and the fatty alcohol is octanol.

12. A hydrophobic antimicrobial solution comprising a tocopherol compound and at least one saturated carboxylic acid with an aliphatic chain of 8-14 carbon atoms, for use in a method of disinfecting an implanted catheter; said method comprising:
introducing said hydrophobic antimicrobial solution to a lumen of a catheter, wherein at least a portion of the catheter is sufficiently porous to permit diffusion of the solution outwardly from the lumen to the outer surface of the catheter and into tissues or the bloodstream surrounding the catheter to inhibit infection;
wherein the acid includes at least one acid selected from the group consisting of octanoic acid, decanoic acid and dodecanoic acid.

## Patentansprüche

1. Kit zum Verschließen eines implantierten Katheters, wobei das Kit Folgendes umfasst:
ein Behältnis, das ein Volumen einer Lösung enthält, umfassend:
a) eine Tocopherol-Verbindung; und
b) mindestens eine gesättigte Carbonsäure mit einer aliphatischen Kette von 8 bis 14 Kohlenstoffatomen; und
ein Lumen eines implantierbaren Katheters, das die Lösung aufnimmt.

2. Kit nach Anspruch 1, wobei die Tocopherol-Verbindung mindestens ein Tocopherol umfasst, das aus der Gruppe ausgewählt ist, bestehend aus α-Tocopherol, Tocopherolacetat und Tocopherolsäure-Succinatester.

3. Kit nach Anspruch 2, wobei die Tocopherol-Verbindung α-Tocopherol ist.

4. Kit nach Anspruch 3, wobei das α-Tocopherol eine Mindestviskosität von 3 Millipascal-Sekunden vor der Kombination mit der mindestens einen Säure aufweist.

5. Kit nach Anspruch 1, wobei die mindestens eine Säure aus der Gruppe ausgewählt ist, bestehend aus Octansäure, Decansäure und Dodecansäure.

6. Kit nach Anspruch 1, ferner umfassend mindestens ein essentielles Öl, wobei das Öl mit einem Fettalkohol gemischt ist, der mindestens einer gesättigten Carbonsäure mit einer aliphatischen Kette von 8 bis 14 Kohlenstoffen entspricht, zur Verbesserung des Aromas der Locklösung.

7. Kit nach Anspruch 6, wobei die mindestens eine Säure Octansäure oder Decansäure oder Dodecansäure oder jedwede Kombination davon ist.

8. Hydrophobe antimikrobielle Katheter-Locklösung, die zur Inhibition einer Infektions- und Blutgerinnselbildung zum Verschließen eines Lumens eines implantierten Katheters konfiguriert ist, der Katheter zu einem Körperlumen eines Patienten hin offen ist, wobei die Lösung Folgendes umfasst:
eine Tocopherol-Verbindung kombiniert mit mindestens einer der folgenden Säuren:
Octansäure, bis zu 50 % (v/v);
Decansäure, bis zu 25 % (v/v); und
Dodecansäure, bis zu 25 % (w/v);
wobei die Kombination des Tocopherols und der mindestens einen Säure eine Viskosität der Lösung von größer als 3 Millipascal-Sekunden bereitstellt.

9. Locklösung nach Anspruch 8, wobei die Tocopherol-Verbindung α-Tocopherol in einer Konzentration von bis zu 50 % (v/v) ist.

10. Locklösung nach Anspruch 8, ferner umfassend mindestens ein essentielles Öl, wobei das Öl mit einem Fettalkohol gemischt ist, der mindestens einer gesättigten Carbonsäure mit einer aliphatischen Kette von 8 bis 14 Kohlenstoffatomen entspricht.

11. Locklösung nach Anspruch 10, wobei das essentielle Öl Orangenöl ist und der Fettalkohol Octanol ist.

12. Hydrophobe antimikrobielle Lösung, umfassend eine Tocopherol-Verbindung und mindestens eine gesättigte Carbonsäure mit einer aliphatischen Kette von 8 bis 14 Kohlenstoffen, zur Verwendung in einem Verfahren zum Desinfizieren eines implantierten Katheters, wobei das Verfahren Folgendes umfasst:
Einbringen der genannten hydrophoben antimikrobiellen Lösung in ein Lumen eines Katheters, wobei mindestens ein Teil des Katheters ausreichend porös ist, um eine Diffusion der Lösung aus dem Lumen nach außen an die äußere Oberfläche des Katheters und in den Katheter umgebende Gewebe oder in den den Katheter umgebenden Blutstrom zur Inhibition einer Infektion zu erlauben;
wobei die Säure mindestens eine Säure einschließt, die aus der Gruppe ausgewählt ist, bestehend aus Octansäure, Decansäure und Dodecansäure.

## Revendications

1. Trousse pour la fermeture d'un cathéter implanté, ladite trousse comprenant :
un récipient contenant un volume d'une solution comprenant :
a) un composé de tocophérol ; et
b) au moins un acide carboxylique saturé ayant une chaîne aliphatique de 8 à 14 atomes de carbone ; et
une lumière de cathéter implantable qui reçoit la solution.

2. Trousse selon la revendication 1, dans laquelle le composé de tocophérol comprend au moins un tocophérol sélectionné dans le groupe constitué de l'alpha-tocophérol, de l'acétate de tocophérol et de l'ester de succinate acide de tocophérol.

3. Trousse selon la revendication 2, dans laquelle le composé de tocophérol est l'alpha-tocophérol.

4. Trousse selon la revendication 3, dans laquelle l'alpha-tocophérol a une viscosité minimum de 3 milliPascal-secondes avant sa combinaison avec ledit au moins un acide.

5. Trousse selon la revendication 1, dans laquelle ledit au moins un acide est sélectionné dans le groupe constitué de l'acide octanoïque, de l'acide décanoïque, et de l'acide dodécanoïque.

6. Trousse selon la revendication 1, comprenant en outre au moins une huile essentielle, dans laquelle l'huile est mélangée avec un alcool gras qui correspond à au moins un acide carboxylique saturé ayant une chaîne aliphatique de 8 à 14 atomes de carbone, pour améliorer l'arôme de la solution verrou.

7. Trousse selon la revendication 6, dans laquelle ledit au moins un acide est l'acide octanoïque ou l'acide décanoïque ou l'acide dodécanoïque ou n'importe quelle combinaison de ceux-ci.

8. Solution verrou pour cathéter, antimicrobienne et hydrophobe, conçue pour inhiber l'infection et la formation de caillots sanguins, destinée à fermer une lumière d'un cathéter implanté, le cathéter débouchant sur une lumière d'un corps d'un patient, la solution comprenant :
un composé de tocophérol combiné avec au moins l'un des acides suivants :
acide octanoïque, jusqu'à 50 % en volume/volume ;
acide décanoïque, jusqu'à 25 % en volume/volume ; et
acide dodécanoïque, jusqu'à 25 % en poids/volume ;
dans laquelle la combinaison du tocophérol et dudit au moins un acide donne à la solution une viscosité supérieure à 3 milliPascal-secondes.

9. Solution verrou selon la revendication 8, dans laquelle le composé de tocophérol est de l'alpha-tocophérol à une concentration jusqu'à 50 % (volume/volume).

10. Solution verrou selon la revendication 8, comprenant en outre au moins une huile essentielle, dans laquelle l'huile est mélangée avec un alcool gras correspondant à au moins un acide carboxylique saturé ayant une chaîne aliphatique de 8 à 14 atomes de carbone.

11. Solution verrou selon la revendication 10, dans laquelle l'huile essentielle est de l'huile d'orange et l'alcool gras est l'octanol.

12. Solution antimicrobienne hydrophobe comprenant un composé de tocophérol et au moins un acide carboxylique saturé ayant une chaîne aliphatique de 8 à 14 atomes de carbone, destinée à une utilisation dans un procédé de désinfection d'un cathéter implanté, ledit procédé comprenant :
l'introduction de ladite solution antimicrobienne hydrophobe dans une lumière d'un cathéter, au moins une partie du cathéter étant suffisamment poreuse pour permettre la diffusion de la solution vers l'extérieur à partir de la lumière jusqu'à la surface externe du cathéter et dans les tissus ou la circulation sanguine entourant le cathéter pour inhiber l'infection ;
l'acide comprenant au moins un acide sélectionné dans le groupe constitué de l'acide octanoïque, de l'acide décanoïque et de l'acide dodécanoïque.
